Europäisches Patentamt

(19) European Patent Office

Office européen des brevets

(11) Numéro de publication: **0 337 898**
**A1**

## (12) DEMANDE DE BREVET EUROPEEN

(21) Numéro de dépôt: **89420097.1**

(22) Date de dépôt: **17.03.89**

(51) Int. Cl.4: **C 08 G 18/82**

(30) Priorité: **22.03.88 FR 8804051**

(43) Date de publication de la demande:
**18.10.89 Bulletin 89/42**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Robin, Jean**
**21, rue Duguesclin**
**F-69006 Lyon (FR)**

**Blind, André**
**10, rue Martin Basse**
**F-69300 Caluire (FR)**

(74) Mandataire: **Vignally, Noel et al**
**Rhône-Poulenc Chimie Service Brevets Chimie Centre**
**de Recherches des Carrières B.P. 62**
**F-69192 Saint-Fons Cédex (FR)**

(54) **Procédé de purification et d'isolement de condensats d'isocyanates par extraction au CO2 liquide ou supercritique.**

(57) La présente invention concerne l'isolement et la purification de condensats d'isocyanates à groupes NCO libres, obtenus à partir de di- ou polyisocyanates aliphatiques, cycloaliphatiques ou arylaliphatiques dont les groupes isocyanates ne sont pas directement liés à un cycle aromatique.

De tels condensats sont utilisés pour préparer des mousses, des élastomères, des adhésifs, des peintures et des vernis, dont les propriétés exceptionnelles sont maintenant bien connues.

Elle comprend essentiellement le traitement par un gaz inerte à l'état liquide ou supercritique desdits condensats bruts à groupes NCO libres.

On obtient ainsi un polyisocyanate contenant une très faible quantité de diisocyanate libre.

EP 0 337 898 A1

Bundesdruckerei Berlin

## Description

## PROCEDE DE PURIFICATION ET D'ISOLEMENT DE CONDENSATS D'ISOCYANATES PAR EXTRACTION AU $CO_2$ LIQUIDE OU SUPERCRITIQUE

La présente invention concerne l'isolement et la purification de condensats d'isocyanates à groupes NCO libres, obtenus à partir de di- ou polyisocyanates aliphatiques, cycloaliphatiques ou arylaliphatiques dont les groupes isocyanates ne sont pas directement liés à un cycle aromatique.

De tels condensats sont utilisés pour préparer des mousses, des élastomères, des adhésifs, des peintures et des vernis, dont les propriétés exceptionnelles sont maintenant bien connues.

Ces condensats à groupes NCO libres sont obtenus en faisant réagir au moins un composé possédant au moins deux groupements réactifs vis à vis des isocyanates, avec un excès molaire de di- ou polyisocyanate, éventuellement au sein d'un solvant inerte vis à vis des groupes NCO. Le composé à groupements réactifs vis à vis des groupes NCO peut comporter comme sites réactifs, des groupements -OH, -SH, COOH, les groupements au sein dudit composé peuvent bien entendu être identiques ou différents. On peut citer comme exemples les diols et les polyols. Le composé portant les groupes réactifs vis-à-vis des groupements NCO peut être constitué par un bi-radical aliphatique, cyclique, cycloaliphatique ou aromatique ; il peut aussi résulter lui-même de la condensation de molécules simples et conduire à un bi-radical comportant, le cas échéant, des hétéroatomes dans la chaîne. De tels condensats peuvent être :

- des polyesters issus de l'estérification d'un ou plusieurs di ou polyols par un ou plusieurs di- ou polyacides, ou encore par réaction d'une lactone cyclique sur une molécule di- ou polyfonctionnelle comportant des groupements -OH, -NH$_2$, -NHR, par exemple ;
- des polyéthers, issus de la condensation d'oxydes cycliques (oxyde d'éthylène, de propylène, de butylène, de tétraméthylène) sur une molécule di- ou polyfonctionnelle comportant des groupes -OH, -NH$_2$ , NH ;
- des condensats mixtes comportant des segments polyéther et polyester.

Ces composés sont mis à réagir avec un excès de di- ou polyisocyanate, éventuellement au sein d'un solvant non réactif avec les groupes isocyanates. Comme di- ou polyisocyanates, on peut citer à titre d'exemples :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,
- le bis(isocyanato-4 cyclohexyl) méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

Ces diisocyanates peuvent être utilisés séparément ou sous forme de mélanges de plusieurs d'entre eux.

Ainsi par exemple, le diisocyanato-1,6 hexane (HDI) qui est l'un des diisocyanates préférés, peut être utilisé seul ou en mélange, notamment avec le diisocyanato-1,5 méthyl-2 pentane et/ou le diisocyanato-1,4 éthyl-2 butane ; des mélanges de ces deux derniers diisocyanates peuvent également être utilisés.

La réaction du polyisocyanate en excès avec le composé antagoniste est réalisée selon les moyens connus, c'est à dire en chauffant le mélange des deux produits, éventuellement en présence d'un catalyseur et/ou d'un solvant.

Le point commun de ces condensats à NCO libres est de renfermer, en fin de réaction, une quantité plus ou moins importante du diisocyanate utilisé en excès. Il est souvent nécessaire d'éliminer ce diisocyanate du polycondensat réalisé, notamment en raison de la toxicité due à la volatilité dudit diisocyanate. En effet, lors de la mise en oeuvre de tels condensats obtenus avec un diisocyanate comme le diisocyanato-1,6 hexane (HDI), la présence d'une quantité plus ou moins importante de HDI peut présenter des risques pour l'utilisation du condensat ; ces risques sont très grands lorsque le condensat est mis en oeuvre en couche mince, comme cela est le cas pour les peintures et vernis, par exemple, car l'émission de vapeurs de HDI toxique peut devenir importante et dépasser les limites autorisées légalement dans l'atmosphère environnante .

Une autre raison peut résider dans la mise en oeuvre de tels condensats, notamment de ceux issus de la réaction de polyesters ou polyéthers avec un excès de diisocyanate (ces condensats étant le plus souvent dénommés prépolymères), pour la fabrication de matériaux élastomériques cellulaires ou non. Dans ce cas, ledit prépolymère est mis à réagir avec une quantité stoechiométrique d'un réactif au moins difonctionnel et comportant des groupes réactifs avec les NCO, au sens indiqué précédemment. Le matériau final comportera une quantité plus ou moins grande du produit de condensation du diisocyanate en excès avec le réactif difonctionnel, dont la présence peut être préjudiciable aux propriétés du matériau élastomérique final. Dans ce cas, l'élimination du diisocyanate libre sera la seule manière d'avoir un prépolymère ayant à la fois un faible degré de condensation et une basse teneur en diisocyanate libre.

L'élimination du diisocyanate en excès peut se faire par des moyens connus comme l'évaporation ou l'extraction par un solvant du diisocyanate, non solvant du condensat. Toutefois, l'évaporation nécessite l'emploi de températures élevées qui sont préjudiciables à la qualité du condensat. Cet inconvénient se manifeste par des réactions secondaires à chaud, conduisant à un produit de viscosité élevée, pouvant provoquer un dépôt dans l'évaporateur ; de plus, un bon épuisement du diisocyanate libre nécessite l'emploi d'évaporateurs à film mince agités ; ce sont des appareils onéreux et qui nécessitent, dans ce cas, des arrêts et nettoyages fréquents, car le plus souvent le condensat fondu se résinifie peu à peu dans l'appareil. D'autre part, l'extraction par un solvant du diisocyanate non solvant du condensat (comme l'hexane, l'octane...) est longue et malaisée ; en effet, dès l'addition du non solvant, le condensat tend à précipiter sous forme d'une masse collante de laquelle il est difficile d'extraire la totalité du diisocyanate monomère libre.

On a trouvé que l'opération de séparation du condensat à NCO libre d'avec l'excès de diisocyanate libre est facile à réaliser par traitement du condensat brut par un gaz inerte à l'état liquide ou supercritique ou par un mélange de tels gaz. Comme gaz inertes, on peut citer à titre d'exemples le gaz carbonique, le butane, l'éthane, le propane, l'éthylène. Le gaz carbonique qui est bon marché, non toxique et ininflammable est le composé dont l'usage est préconisé. On peut également utiliser le gaz carbonique comme fluide extractant, de préférence à l'état supercritique, associé à un autre gaz inerte (coextractant) à l'état liquide ou supercritique, tel que notamment le propane, l'éthane ou le butane. On a constaté que, non seulement le $CO_2$ liquide ou supercritique dissout et extrait le diisocyanate libre présent dans le condensat, mais aussi que le condensat, au sein du $CO_2$ liquide ou supercritique, reste liquide quand bien même il a une consistance solide ou résineuse à la températaure d'extraction, ce qui permet ensuite de véhiculer et de dissoudre commodément, et à température modérée, le condensat débarrassé de son diisocyanate libre.

Cette extraction peut se faire en continu ou en discontinu à une température supérieure à 31,4°C (température critique du $CO_2$) et à une pression comprise entre 7,3 et 50 MPa (73 et 500 bar). De préférence, la température est comprise entre 31,4°C et 100°C et la pression entre 7,3 et 35 MPa (73 et 350 bar). Ceci concerne le $CO_2$ à l'état supercritique. A l'état liquide, le $CO_2$ est mis en oeuvre à une température comprise entre 0 et 31°C, et sous une pression de 3 à 50 MPa (30 à 500 bar). Il est souvent préférable d'opérer entre 20 et 31°C lorsque la viscosité du condensat est important. La pression peut aller de 6 à 30 MPa (60 à 300 bar).

Le condensat renfermant le diisocyanate libre est traité par le $CO_2$ liquide ou à l'état supercritique, éventuellement en mélange avec un coextractant ; on peut opérer en discontinu, c'est à dire en mélangeant dans un réacteur le condensat à NCO libre à purifier avec le $CO_2$ à l'état liquide ou supercritique (et avec l'éventuel coextractant).

Il est possible de traiter la masse réactionelle dès la fin de la réaction de condensation.

Cependant, compte tenu des quantités importantes de diisocyanate monomère en excès et le plus fréquemment du ou des solvants, il peut être préférable et plus économique de procéder préalablement à une élimination rapide de la plus grande partie du diisocyanate libre en excès et du solvant lorsque ce dernier est présent.

Ce traitement, qui est souvent une évaporation très rapide sous pression réduite, ne nécessite pas de chauffer à une température élevée. Il ne présente donc pas, ou très peu, l'inconvénient mentionné précédemment, c'est-à-dire de provoquer dans l'appareillage une dépôt polymérique nécessitant de fréquents arrêts pour nettoyage.

En pratique donc, la masse obtenue après la réaction de condensation est envoyée dans un évaporateur à couche mince, qui permet de séparer la majeure partie du solvant et une partie notable du diisocyanate monomère en excès.

Le polyisocyanate obtenu, contenant une quantité encore relativement importante de diisocyanate libre et éventuellement des traces de solvant, est alors traité à l'aide de $CO_2$ liquide ou de $CO_2$ à l'état supercritique (et d'un éventuel coextractant).

Après séparation du gaz carbonique renfermant le diisocyanate libre et éventuellement du solvant, d'avec le condensat purifié, on peut séparer le $CO_2$ (et l'éventuel coextractant) d'avec les produits extraits par détente et/ou augmentation de température.

L'extraction peut être conduite de manière conventionnelle, dans un appareillage connu en soi.

A partir d'une source de gaz d'extraction (le plus souvent le gaz carbonique) on envoie ledit gaz dans un échangeur thermique où il est liquéfié ; il est ensuite véhiculé à la pression désirée à l'aide d'une pompe vers un autre échangeur thermique où il est porté à la température choisie pour l'extraction.

Le gaz d'extraction à l'état liquide ou supercritique est ensuite envoyé dans l'appareil d'extraction qui peut être par exemple une colonne remplie d'un garnissage permettant un meilleur contact entre le condensat avec le gaz d'extraction. Le condensat peut être introduit à l'autre extrémité de la colonne : on a alors une extraction à contre-courant. Moins fréquemment, on peut l'introduire à la même extrémité que le gaz d'extraction : on a alors une extraction à co-courant. Le condensat purifié est récupéré à une extrémité de la colonne d'extraction, tandis que le gaz d'extraction chargé du diisocyanate libre, et éventuellement de solvants, est traité pour le séparer des produits extraits.

On peut pour cela agir soit par diminution de sa pression soit par augmentation de sa température, soit à la fois par diminution de sa pression et augmentation de sa température. Ces conditions ont pour but de modifier le pouvoir solvant du gaz d'extraction.

La diminution de pression ou détente, peut se faire en une ou plusieurs étapes et le gaz d'extraction peut être détendu jusqu'à une pression égale à la pression atmosphérique ou jusqu'à une pression plus élevée, sous laquelle il sera recyclé dans le cas d'un procédé continu.

En effet, si le gaz d'extraction est recyclé, il est économiquement préférable de ne pas le détendre jusqu'à la pression atmosphérique, ce qui nécessiterait une plus grande dépense d'énergie pour le recomprimer dans le cycle suivant le procédé. Il est préférable de le détendre jusqu'à une pression sous laquelle les composés extraits ne sont pas, ou sont très peu, solubles.

Le condensat, liquéfié par le gaz d'extraction sous pression peut être véhiculé et détendu dans un récipient de capacité suffisante dans lequel le gaz détendu pourra s'échapper, laissant sur les parois le condensat sous forme de poudre ou de liquide visqueux ; ou encore, le condensat liquéfié par le gaz d'extraction peut être reçu dans un solvant ou dans un mélange de solvants ce qui donne après détente, une solution de condensat prête à l'emploi.

Comme cela a été indiqué précédemment, le procédé peut être mis en oeuvre de manière continue ou discontinue, et l'appareillage utilisé n'est pas limité à la description du principe de fonctionnement décrit ci-avant.

Généralement, dans le cadre du procédé de l'invention on préfère mettre en oeuvre le gaz d'extraction à l'état supercritique.

Les exemples qui suivent illustrent la présente invention.

EXEMPLE 1 :

Dans un réacteur tricol de 1 000 cm³, muni d'une agitation, d'un chauffage par bain d'huile, on charge 200g de polyester, qui est un polyadipate d'éthylène glycol, à terminaisons hydroxylées, de masse moléculaire 2000 (présentant un indice d'acide de 2 et un acide d'hydroxyle de 56).

On fond le produit par chauffage à 110°C sous courant d'azote. On arrête l'azote et on met l'appareil sous pression réduite (1330 à 2000 Pa) pendant 1 heure sous agitation.

On remet l'appareil sous pression atmosphérique par introduction d'azote sec, on refroidit à 80°C et on coule 33,6 g de diisocyanato-1,6 hexane (HDI). La température s'élève peu à peu et on la maintient à 110°C pendant 2 heures.

Le prépolymère ainsi obtenu titre 3,2 % en poids de groupements NCO et renferme 3,5 % en poids de HDI libre.

Dans un réacteur de 45 cm³ résistant à la pression, on charge 20 g du prépolymèe obtenu précédemment que l'on fond à 80°C.

En maintenant la température à 80°C, on introduit le $CO_2$ jusqu'à une pression de 14 MPa, maintenue au moyen d'un régulateur de pression.

Quand le système est en équilibre, on envoie par le bas du réacteur le $CO_2$ avec un débit de 500 g/heure.

Après 2 heures d'extraction la quantité extraite est de 0,8 g (après élimination du $CO_2$) dont 0,6 g de HDI.

Le prépolymère restant ne renferme plus que 0,05 % en poids de HDI.

EXEMPLE 2

On prépare un prépolymère comme décrit dans l'example 1, mais en remplaçant le polyester par un polyéther (polytétrahydrofuranne de masse moléculaire 2 000, vendu par la Société DU PONT sous le nom de TERATHANE 2 000).

Le prépolymère ainsi obtenu titre 3,8 % en poids de groupements NCO et renferme une teneur de 3,7 % en poids de HDI libre.

Dans le réacteur décrit dans l'exemple 1, on traite 20 g de ce prépolymère par du $CO_2$ à l'état supercritique, pendant 2 heures à 60°C sous 18,5 MPa.

On extrait ainsi 0,7 g de HDI et le prépolymère traité ne renferme plus que 0,03 % en poids de HDI libre.

**Revendications**

1°) Procédé de purification et d'isolement de condensats d'isocyanates à groupes NCO libres obtenus à partir de di- ou polyisocyanates aliphatiques, cycloaliphatiques et/ou arylaliphatiques en excès et d'au moins un composé antagoniste possédant au moins deux groupements réactifs vis-à-vis des groupes NCO, caractérisé en ce que lesdits condensats bruts et à groupes NCO libres sont traités par un gaz inerte à l'état liquide ou supercritique.

2°) Procédé selon la revendication 1, caractérisé en ce que le condensat est préparé à partir d'au moins un diisocyanate monomère choisi dans le groupe comprenant :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,
- le bis(isocyanato-4 cyclohexyl) méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

3°) Procédé selon la revendication 1 ou 2, caractérisé en ce que le condensat est préparé à partir de diisocyanato-1,6 hexane seul ou en mélanges.

4°) Procédé selon l'une quelconque des revendications 1 à 3, caractérisé en ce que le composé antagoniste utilisé pour préparer le condensat à groupes NCO libres est lui-même un condensat du type des polyesters, du type des polyéthers ou de type mixte polyéther-polyester.

5°) Procédé selon l'une des revendications 1 à 4, caractérisé en ce que le gaz inerte utilisé à l'état liquide ou supercritique est le gaz carbonique.

6°) Procédé selon l'une des revendications 1 à 4, caractérisé en ce que le gaz inerte utilisé est le gaz carbonique, de préférence à l'état supercritique, associé à un autre gaz inerte (coextractant) à l'état liquide ou supercritique, tel que le propane, l'éthane ou le butane.

7°) Procédé selon la revendication 5, caractérisé en ce que l'on opère à une température comprise entre 0 et 31°C et sous une pression de 3 à 50 MPa.

8°) Procédé selon l'une des revendications 5 ou 7, caractérisé en ce que l'on opère à une température comprise entre 20 et 31°C et sous une pression de 6 à 30 MPa.

9°) Procédé selon l'une des revendications 5 ou 6, caractérisé en ce que l'on opère à une température supérieure à la température critique du gaz carbonique de préférence inférieure à 100°C et sous une pression de 7,3 à 50 MPa et de préférence de 7,3 à 35 MPa.

10°) Procédé selon l'une des revendications 1 à 9, caractérisé en ce que l'on élimine, préalablement à l'extraction par le gaz inerte à l'état liquide ou supercritique, la plus grande partie de l'excés de diisocyanate monomère et du solvant lorsque ce dernier est présent.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 105 242  (MOBAY CHEMICAL CORPORATION) * page 1 - page 2, ligne 16; page 3, lignes 17-27; page 4, lignes 23-27; page 5, lignes 10-14; exemples 6,7; revendications 1,4,7,8 * | 1-4 | C 08 G   18/82 B 01 D   11/04 |
| A | EP-A-0 052 276  (E.I. DU PONT DE NEMOURS AND COMPANY) * page 1, lignes 5-12; page 2, lignes 12-18; exemple; revendications 1-8 * | 1,5-10 | |
| A | US-A-4 703 105  (S.R. ALLADA) * abrégé; colonne 4, lignes 32-60; exemples; revendications 1,5-8 * | 1,5-10 | |
| A | J.H. SAUNDERS et al.:"POLYURETHANES; CHEMISTRY AND TECHNOLOGY" 3ième édition, partie II:"Technology", pages466-4 68, 1983, R.E. KRIEGER PUBLISHING CORP. Malabar, US * le document en entier * | 1 | |

|  |  |  | DOMAINES TECHNIQUES RECHERCHES (Int. Cl.4) |
|---|---|---|---|
|  |  |  | B 01 D   11/00 C 08 G   18/00 |

Le présent rapport a été établi pour toutes les revendications

| Lieu de la recherche | Date d'achèvement de la recherche | Examinateur |
|---|---|---|
| BERLIN | 19-06-1989 | HOEPFNER W.W.G. |